# EUROPEAN PATENT SPECIFICATION

(11) **EP 3 307 177 B1**
(45) Date of publication and mention of the grant of the patent: **29.05.2019**
(21) Application number: 16731439.2
(22) Date of filing: 07.06.2016
(51) Int. Cl.: A61B 10/02, A61B 10/04

(54) **FLEXIBLE BIOPSY NEEDLE**
FLEXIBLE BIOPSIENADEL
AIGUILLE DE BIOPSIE SOUPLE

(30) Priority: 11.06.2015 US 201562174214 P
(43) Date of publication of application: 18.04.2018
(73) Proprietor: Boston Scientific Scimed, Inc., Maple Grove, MN 55311-1566 (US)
(72) Inventor: ROHL, James P., St. Paul, Minnesota 55112 (US); ECKERLINE, Katharine, Boston, Massachusetts 02113 (US); FORSYTH, Bruce R., Hanover, Massachusetts 55341 (US); RUNDQUIST, Charles, White Bear Lake, Minnesota 55110 (US); WULFMAN, David Robert, Minneapolis, Minnesota 55405 (US); OTT, Jason, Brooklyn Park, Minnesota 55443 (US); LEHSE, David John, Oakdale, Minnesota 55128 (US)
(74) Representative: Vossius & Partner Patentanwälte Rechtsanwälte mbB
(86) International application number: PCT/US2016/036218
(87) International publication number: WO 2016/200811

(56) References cited:
- WO-A1-2006/065913
- WO-A1-2012/015801
- WO-A1-2015/089372
- WO-A2-01/74252
- US-A1- 2014 276 051

## Description

### Background

Needle biopsy procedures are common for the diagnosis and the staging of disease. For example, a fine needle aspiration needle may be advanced through a working channel of an endoscope to a target tissue site. In some cases, the needle must be navigated along a tortuous path through a body lumen to reach target tissue. Thus, the needle must have sufficient flexibility to be inserted to the target tissue while also retaining sufficient strength to penetrate target tissue to collect a tissue sample.

### Summary

The present disclosure relates to a needle device for obtaining a tissue sample, comprising a distal member extending longitudinally from a proximal end to a distal end and including a distal channel extending longitudinally therethrough, the distal member formed of a first material selected to permit flexing of the distal member, a proximal member extending longitudinally from a proximal end to a distal end and including a proximal channel extending longitudinally therethrough, the proximal member formed of a second material selected to provide axial and torsional strength therealong, and a coupler for connecting the distal member and the proximal member, the coupler extending longitudinally from a proximal end configured to engage the distal end of the proximal member to a distal end configured to engage the proximal end of the distal member.

In an embodiment, the distal member may include a plurality of slots extending laterally thereinto form an exterior surface thereof to an interior surface of the distal channel, the plurality of slots positioned therealong to permit a desired flexing thereof.

In an embodiment, the distal member may be at least partially formed of Nitinol.

In an embodiment, the proximal member may be formed of stainless steel.

In an embodiment, the distal member may include an outer sleeve and an inner sleeve, the inner sleeve press fit into the outer sleeve to support the outer sleeve during a flexing thereof.

In an embodiment, the outer sleeve may be formed of stainless steel and the inner sleeve may be formed of Nitinol.

In an embodiment, the proximal end of the coupler may be configured to be received within the proximal channel of the proximal member.

In an embodiment, the distal end of the coupler may be configured to be mounted over the proximal end of the distal member.

In an embodiment, the coupler may be welded to the distal and proximal members.

In an embodiment, the device further comprises a filter positionable within a coupler channel proximally of the proximal end of the distal member to prevent passage of a tissue sample proximally therepast while permitting passage therethrough of one of a vacuum force and a flushing fluid.

In an embodiment, the filter may be formed via one of molding valves, spinning fibers, blown fibers and ceramics.

In an embodiment, a central portion of the filter may configured to receive a flushing fluid therethrough and radially extending outer portions may be configured to receive a vacuum force therethrough.

The present disclosure also relates to a device for collecting a tissue sample, comprising a delivery catheter extending along a longitudinal axis from a proximal end to a distal end and including a lumen extending longitudinally therethrough, a central axis of the lumen offset from the longitudinal axis so that a first portion of a wall along a first longitudinal side of the delivery catheter has a smaller thickness than a second portion of the wall along a second longitudinal side of the delivery catheter opposing the first side and a needle extending longitudinally from a proximal end to a distal tapered tip and including a channel extending therethrough, the needle including a flat extending along at least a portion of a length of an exterior surface thereof, the needle slidably received within the lumen of the delivery catheter so that the flat faces the first side of the delivery catheter.

In an embodiment, the flat may extend to a distal-most end the distal tapered tip.

In an embodiment, the delivery catheter may include a corresponding flat extending along a length of an interior surface of the delivery catheter along the first side thereof.

The present disclosure also relates to a method for assembling a needle device for collecting a tissue sample, comprising engaging a proximal end of a distal member with a distal end of a coupler, the distal member extending longitudinally from the proximal end to the distal end and including a distal channel extending therethrough, the distal member formed of a first material permitting a flexing thereof and engaging a distal end of a proximal member with a proximal end of a coupler, the proximal member extending longitudinally from the proximal end to the distal end and including a proximal channel extending therethrough, the proximal member formed of a second material providing one of axial and torsional strength thereof.

In an embodiment, the method may further comprise forming a plurality of slots extending laterally into the distal member via one of laser cutting, wire electrical discharge machining and stamping.

In an embodiment, the distal end of the coupler may be mounted over the proximal end of the distal member while the proximal end of the coupler is received within the proximal channel so that a coupler channel, the distal channel and the proximal channel are substantially axially aligned.

In an embodiment, the method may further comprise welding the coupler to the distal and proximal channels via a first longitudinal slot extending along the distal end of the coupler and a second longitudinal slot extending along the distal end of the proximal member.

In an embodiment, the method may further comprise positioning a filter within the coupler proximally of the proximal end of the distal member to prevent a collected tissue sample from moving proximally therepast while permitting one of a flushing fluid and a vacuum force to be passed therethrough.

WO 2006/065913 describes a needle device according to the preamble of claim 1.

### Brief Description

Fig. 1 shows a perspective view of a portion of a device according to an exemplary embodiment of the present disclosure;
Fig. 2 shows a longitudinal cross-sectional view of a portion of the device of Fig. 1;
Fig. 3 shows a longitudinal cross-sectional view of a distal tip of the device of Fig. 1;
Fig. 4 shows a longitudinal cross-sectional view of a portion of a distal member of the device of Fig. 1;
Fig. 5 shows a longitudinal cross-section view of a device according to a further embodiment of the present disclosure;
Fig. 6 shows a longitudinal cross-sectional view of a filter of Fig. 5, in a first configuration;
Fig. 7 shows a longitudinal side view of the filter of Fig. 5, in the first configuration;
Fig. 8 shows a longitudinal cross-sectional view of the filter of Fig. 5, in a second configuration;
Fig. 9 shows a longitudinal side view of the filter of Fig. 5, in the second configuration;
Fig. 10 shows a perspective view of a filter according to an alternate embodiment of the present disclosure;
Fig. 11 shows a perspective view of a distal portion of a device according to another embodiment of the present disclosure;
Fig. 12 shows a lateral cross-sectional view of the device of Fig. 11;
Fig. 13 shows a perspective view of a distal portion of a device according to another exemplary embodiment of the present disclosure; and
Fig. 14 shows a lateral cross-sectional view of a device according to yet another exemplary embodiment of the present disclosure.

### Detailed Description

The present disclosure may be further understood with reference to the following description and the appended drawings, wherein like elements are referred to with the same reference numerals. The present disclosure relates to biopsy devices and, in particular, to biopsy needles for collecting tissue samples. Exemplary embodiments of the present disclosure describe a needle formed of separate proximal and distal members coupled to one another via a coupler. This permits the proximal and distal members to be formed of different materials that may be selected to obtain desired characteristics for each of the proximal and distal members. The needle may further include a filter positioned within the coupler to maintain a collected tissue sample within the distal member of the needle while permitting fluids to pass into the proximal member. It should be noted that the terms "proximal" and "distal" as used herein refer to a direction toward (proximal) and away from (distal) a user of the device.

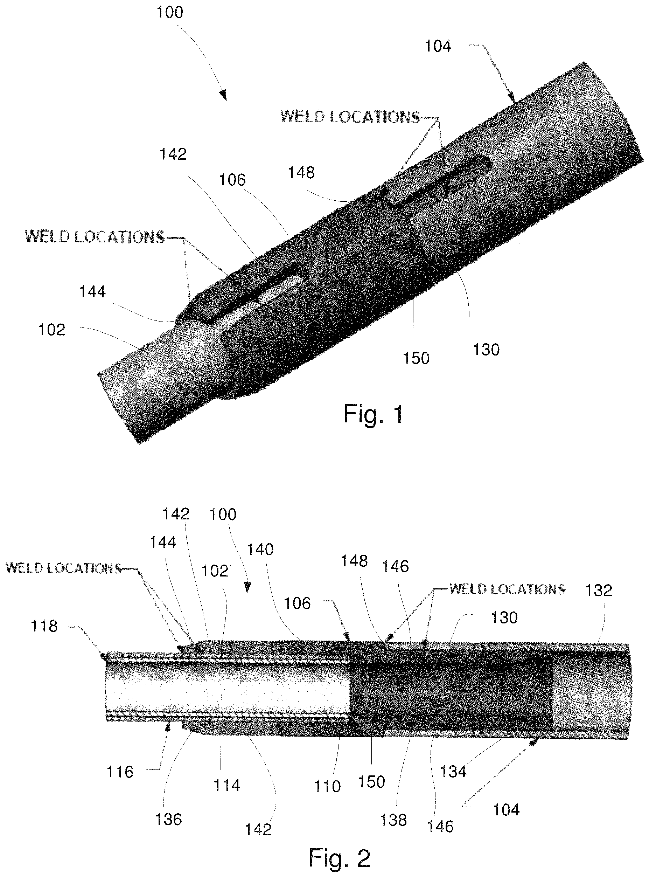

As shown in Figs. 1 - 3, a needle device 100 according to an exemplary embodiment of the present disclosure comprises a distal member 102 and a proximal member 104 connected to one another via a coupler 106. As described above, the distal member 102 may be formed of a different material and/or may be formed with a size that differs from the proximal member 104 as properties desired for the distal member 102 will differ from those desired for the proximal member 104 and the use of separate distal and proximal members may be selected to have desired characteristics. For example, in one exemplary embodiment, a material of the distal member 102 may be selected for higher flexibility as the needle device 100 is inserted through a tortuous path (e.g., through a body lumen) to reach target tissue within a living body. A material of the proximal member 104 may then be selected to exhibit increased axial strength to permit the needle device 100 to be inserted into the target tissue. Although the increased axial strength of the material of the proximal portion 104 may be less flexible than that of the distal portion 102, those skilled in the art will recognize that the distal portion of such a device is generally required to be bendable about a smaller radius to traverse the path along which the distal end of an endoscope or other insertion device is steered to a target site.

The distal member 102 extends longitudinally from a distal member proximal end 110 to a distal end 112 and includes a distal channel 114 extending therethrough from the proximal end 110 to the distal end 112. The distal end 112 according to this embodiment includes a sharp, tissue piercing tip 124 to facilitate penetration of target tissue. For example, all or a portion of the distal member 102 may be formed of Nitinol or other material capable of flexing sufficiently during insertion into a living body (e.g., to permit insertion along a tortuous path such as through a natural body lumen without damaging the surrounding tissue). In one exemplary embodiment, the entire distal member 102 may be formed of Nitinol while, in another exemplary embodiment, the distal member 102 includes an outer sleeve 116 of stainless steel formed about an inner sleeve 118 of Nitinol. The inner sleeve 118 may, for example, be press fit into the outer sleeve 116. The Nitinol inner sleeve 118 may help support an inelastic material such as, for example, the stainless steel outer sleeve 16, to resist kinking or fatigue fracturing during cyclic bending. The inner sleeve 118 and the outer sleeve 116 may have the same or varying lengths. In one embodiment, the inner sleeve 118 may extend along only a portion of a length of the outer sleeve 116 that is required to flex about a small bending radius. Although the distal member 102 is described as comprising an inner and outer sleeve 118, 116, the distal member 102 may alternatively be formed of a single tubular member.

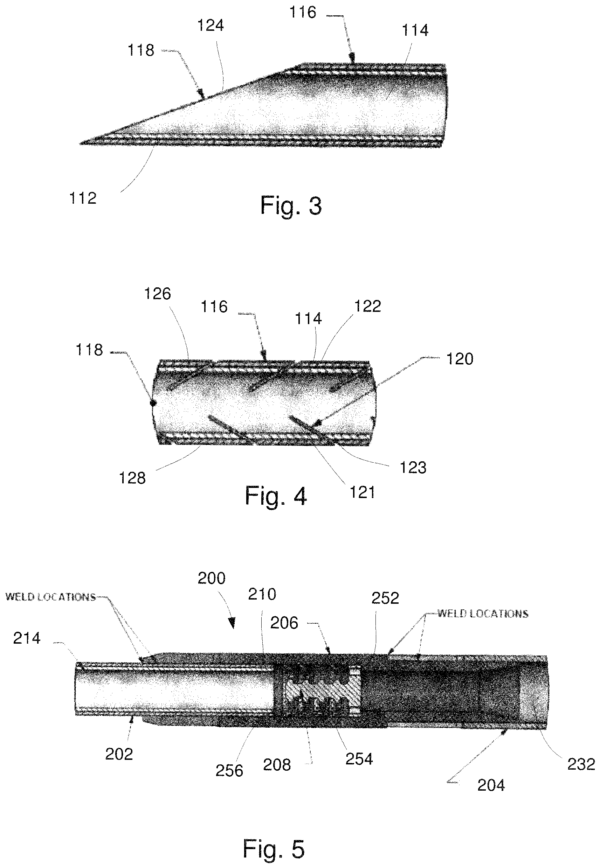

As shown in Fig. 4, the distal member 102 may further include a plurality of slots 120 extending laterally thereinto to enhance the flexibility thereof. In particular, each of the slots 120 extend radially into the distal member 102 from an exterior surface 122 thereof to open into the channel 114. In one exemplary embodiment, each of the slots 120 extends in a plane substantially perpendicular to a longitudinal axis of the distal member 102. In another embodiment, each of the slots 120 extends along a path that is angled relative to a plane perpendicular to the longitudinal axis of the distal member 102. In particular, each slot 120 may extend in a plane angled with respect to the longitudinal axis of the distal member 102 with ends 121 of the slot 120 further proximally than a midpoint 123 of the slot 120. For example, the slots 120 may be formed on a first side 126 of the distal member 102 to enhance a flexibility of the distal member 102 in a direction toward the midpoints 123 of the slots 120. In another example, the slots 120 are also formed on a second side 128 of the distal member 102 substantially opposing the first side 126 so that a flexibility of the distal member 102 in two opposing directions may be facilitated. The slots 120 may be formed via one of laser cutting, wire electrical discharge machining (EDM) and stamping.

The proximal member 104 extends longitudinally from a proximal member proximal end (not shown) to a proximal member distal end 130 and includes a proximal channel 132 extending therethrough from the proximal member proximal end to the proximal member distal end 130. The proximal member 104 according to this embodiment is formed of a material selected to enhance an axial and/or torsional strength of the proximal member 104 to facilitate pushing the needle device 100 through the body along the tortuous path and to then penetrate the target tissue. For example, the proximal member 104 of this embodiment is formed of stainless steel. Forming the proximal member 104 of stainless steel rather than having a single needle formed entirely of, for example, Nitinol, may reduce manufacturing costs. The stainless steel of the proximal member 104 allows the distal member 102 to maintain an axial and/or torsional strength while the distal member 102 flexes to navigate the needle device 100 through even tortuous paths of the body. In addition, the proximal member 104 and the distal member 102 may have varying sizes. The proximal member 104 may, for example, have a size ranging from between 17 and 22 gauge (e.g., outer diameter ranging from between about 0.7176 to 1.473 mm and inner diameter ranging from between about 0.1524 to 0.203 mm) while the distal member 102 may have a size ranging from between 22 and 27 gauge (e.g., outer diameter ranging from between about 0.4128 to 0.7176 mm and inner diameter ranging from between about 0.1016 to 0.1524 mm). The proximal member 104 may have a constant diameter along a length thereof. The distal member 102 may also have a constant diameter along a length thereof or alternatively, may taper along at least a portion of a length thereof. The distal member 102 may have a tip that is mechanically tapered or machine tapered along an interior or exterior. A tapering along an interior of the tip of the distal member 102 may aid in tissue collection therein.

The distal member 102 and the proximal member 104 may be connected to one another via the coupler 106, which extends longitudinally from a coupler proximal end 134 to a coupler distal end 136 and includes a coupler channel 138 extending therethrough. Similarly to the proximal member 104, the coupler 106 may be formed of a material selected to enhance a strength of the needle device 100 such as, stainless steel. The coupler distal end 136 is configured to engage the distal member proximal end 110 while the coupler proximal end 134 is configured to engage the proximal member distal end 130. In one exemplary embodiment, coupler channel 138 at the coupler distal end 136 is sized and shaped to receive the distal member proximal end 110 therein. The coupler proximal end 134 is sized and shaped to be received within the proximal channel 132 at the proximal member distal end 130. The coupler proximal end 134 in this embodiment has a cross-sectional area (e.g., diameter) smaller than a cross-sectional area of a distally extending portion 140 thereof such that, when the coupler proximal end 134 is received within the proximal channel 132, an exterior surface of the proximal member 104 is flush with an exterior surface of the coupler 106. In other words, the distally extending portion 140 of the coupler 106 has an outer diameter substantially the same as an outer diameter of the proximal member 106.

The coupler 106 may further include at least one longitudinal slot 142 extending proximally from the coupler distal end 136 so that, when the distal member proximal end 110 is received within the coupler 106, the coupler 106 may be welded to the distal member 102 at the location of the longitudinal slot 142 and/or at a point where a distal edge 144 of the coupler 106 contacts the exterior surface 122 of the distal member 102. In one exemplary embodiment, the coupler 106 includes a pair of longitudinal slots 142, each of the longitudinal slots 142 extending proximally along substantially diametrically opposing portions of the coupler 106. In addition, the proximal member 104 may include at least one longitudinal slot 146 extending proximally from the proximal member distal end 130 so that, when the proximal member distal end 130 receives the proximal end 134 of the coupler 106 therein, the coupler 106 and the proximal member 104 may be welded together at the location of the longitudinal slot 146 and/or at a point where a distal edge 148 of the proximal member 104 contacts a coupler proximal end 150 of the distally extending portion 140. In one exemplary embodiment, the proximal member 104 may include a pair of longitudinal slots 146, each of the longitudinal slots 146 extending along diametrically opposing portions thereof.

Although the exemplary embodiment describes the coupler 106 as welded to the distal and proximal members 102, 104, the distal and proximal member 102, 104 may be fixed to the coupler 106 in any of a variety of ways. For example, adhesive may be applied to the longitudinal slots 142, 146. In another example, the coupler 106 may engage the distal member 102 and the proximal member 104 via a friction fit. In addition, although the exemplary embodiment describes the proximal end 134 as received within the proximal channel 132 and the distal end 136 as extending over the proximal end 110 of the distal member 102, the coupler 106 may be used to connect the distal and proximal member 102, 104 together in any of a variety of ways. For example, the proximal end 134 may extend about the proximal member 104 while the distal end 136 is received within the distal channel 114. In another example, both the distal and proximal members 102, 104 may be received within the coupler channel 138.

The coupler 106 according to this embodiment connects the distal member 102 and the proximal member 106 so that the distal channel 114, the proximal channel 132 and the coupler channel 138 are all axially aligned and open to one another. Once the needle device 100 has been assembled, as described above, the needle device 100 may be inserted into the living body until the distal end 112 of the distal member 102 reaches the target tissue. The distal end 112 may then be inserted into the target tissue so that a tissue sample may be collected within the distal channel 114.

According to a further embodiment, a needle device 200, as shown in Figs. 5 - 9, may be substantially similar to the needle device 100 described above, comprising a distal member 202 and a proximal member 204 connected to one another via a coupler 206. The needle device 200, however, further comprises a filter 208 housed within the coupler 206 between the distal and proximal members 202, 204 to prevent tissue collected within a distal channel 214 of the distal member 202 from moving proximally therepast into a proximal channel 232 of the proximal member 204 while permitting fluid to be drawn proximally therethrough (e.g., under suction) or flushed distally therepast to flush fluid out of the needle device 100.

As shown in Figs. 5, the filter 208 may be positioned in the coupler 206, proximally of a proximal end 210 of the distal member 202. A shoulder 252 within the coupler 206 prevents the filter 208 from moving proximally therepast such that the filter 208 is fixed between the proximal end 210 of the distal member 202 and the shoulder 252. A portion of the coupler 206 received within the proximal channel 232 or otherwise coupled to the proximal member 204 extends proximally of the shoulder 252.

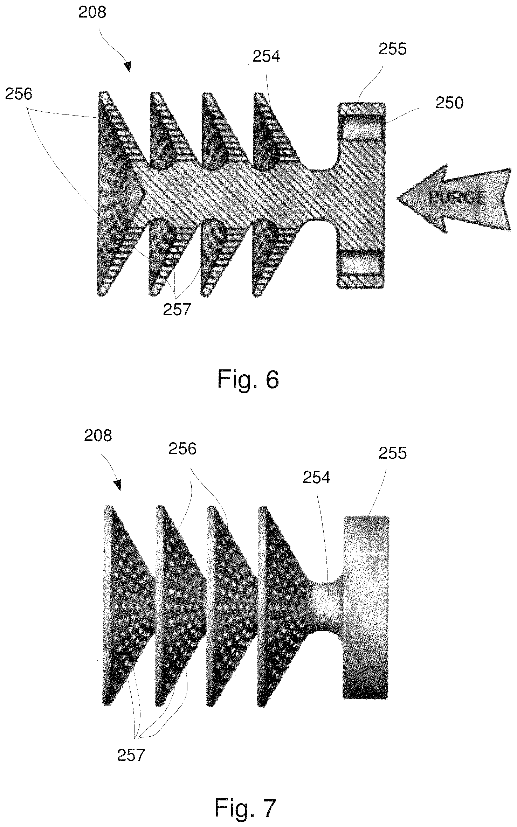

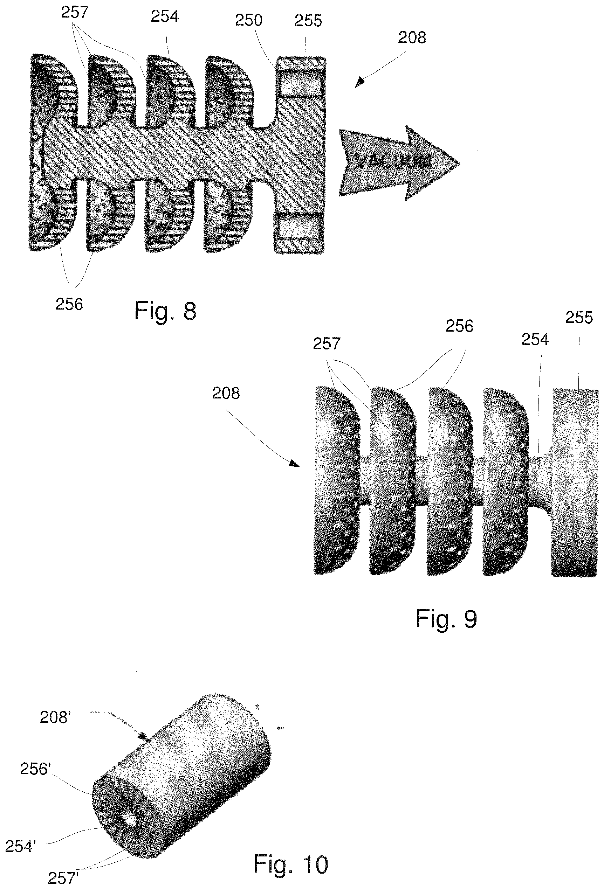

The filter 208 is configured so that a flushing solution or fluid such as, for example, saline, may be passed distally therethrough, as shown in Fig. 6 - 7, to flush a collected tissue sample from the distal channel 214. The filter 208 is configured so that a vacuum force may be applied therethrough, as shown in Figs. 8 - 9, to aid in collecting the tissue sample into the distal channel 214 while preventing the tissue sample from passing proximally therepast so that the collected tissue sample is maintained in a position within the distal channel 214. The filter 208 includes a central portion 254 and an extending portion 256 extending radially therefrom. The extending portion 256 may includes layers of membranes. The filter 208 may also include a flange 255 at a proximal end of the central portion 254. The filter 208 may include a molding valve, espinning fibers, blown fibers, and ceramics. For example, in the embodiment shown, the filter 208 is formed of spinning fibers. In particular, as shown in Figs. 8 - 9, fibers of the radially extending portion 256 cause the radially extending portion 256 to deform as a vacuum force is applied therethrough.

The extending portion 256 of the filter 208 may be formed of a membrane material having pores 257 sized to allow a vacuum force to pass therethrough, but which prevent large tissue and cells from staying trapped distally in the distal channel 114. The extending portion 256 may be formed of a material such as, for example, PTFE, PVDF-HFP, etc. A material of the extending portion 256 may be manufactured using conventional methods to make filtration media such as espin, cryo stretching and chemical etching. The layers and/or membranes of the extending portion 256 may be captured on or in the central portion 254, which may be configured as a molded silicon body. The layers of membranes are protecting by the tooling during the molding.

A vaccum force may be applied through a hole 250 extending through the flange 255 to draw a tissue sample into the distal channel 214, while separating blood cells from the tissue through the filter. After the sample has been acquired, the collected tissue may be purged using saline applied through a separate hole which may extend through the central portion 254. The vaccum force and the saline may be applied through separate holes to prevent mixing of blood cells and undesired fluids.

According to an alternate embodiment, as shown in Fig. 10, a filter 208', which is substantially similar to the filter 208, is formed of porous ceramics or sintered metals such as almunium. Similarly to the filter 208, a central portion 254' of the filter 208' is configured to permit a fluid to be passed distally therethrough while a radially surrounding portion 256' permits vacuum force to be drawn proximally therethrough. The central portion 254' is configured as a lumen extending longitudinally therethrough while the radially surrounding portion 256' includes pores 257' through which the vacuum force may be applied. A collected tissue sample may be purged distally via, for example, a saline solution passed through the central portion 254 so that blood cells are sorted from the collected tissue. The filter 208' may be used in the needle device 200, in substantially the same manner as the filter 208.

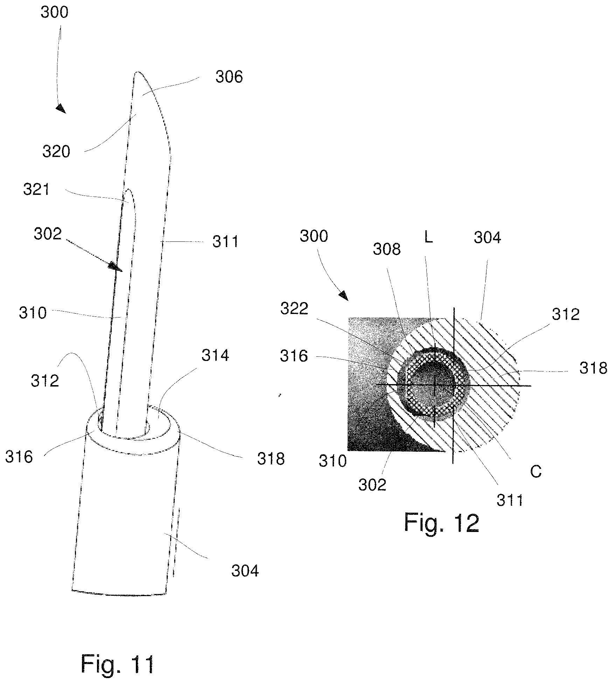

As shown in Figs. 11 - 12, a device 300 according to another exemplary embodiment of the present disclosure comprises a needle 302 passed through a delivery catheter 304 to a target area within a living body. In some cases, however, especially when the device 300 is being inserted to the target area via tortuous paths of a body lumen, a sharp tip 306 of the needle 302 may pierce a material of the delivery catheter 304, clogging a channel 308 of the needle 302 and/or causing the needle 302 to become stuck in the material of the delivery catheter 304. The needle 302 and the delivery catheter 304 include corresponding aligning features which prevent the tip 306 of the needle 302 from piercing the delivery catheter 304. In particular, the needle 302 may include a flat 310 which is in a fixed orientation relative to an offset lumen 312 of the delivery catheter 304.

The delivery catheter 304 is formed of flexible material extending along a longitudinal axis L from a proximal end to a distal end 314. The lumen 312 extends longitudinally through the delivery catheter 304 offset from the longitudinal axis L. In other words, a central axis C along which the lumen 312 extends is not coaxial with the longitudinal axis L and may extend substantially parallel thereto such that a thickness of a wall 314 of the delivery catheter 304 along a first longitudinal side 316 of the delivery catheter 304 is smaller than a thickness of the wall 314 along a second longitudinal side 318 of the delivery catheter diametrically opposing the first side 316. Thus, the delivery catheter 304 is more likely to flex toward the first side 316 thereof.

The needle 302 extends longitudinally from a proximal end to a distal end 320, which includes a tissue penetrating distal tip 306. The channel 308 extends longitudinally through the needle 302 from the proximal to the distal end 320. The flat 310 extends along at least a portion of a length of an exterior surface 311 of the needle 302 in longitudinal alignment with the tip 306. In one exemplary embodiment, as shown in Fig. 11, a distal end 321 of the flat 310 is separated from the tip 306 by a small distance. For example, the flat 310 may be separated from the tip 306 by a distance ranging between 0.1 mm and 1.0 mm. The flat 310 reduces a thickness of a wall 324 along the flat 310 so that, similarly to the delivery catheter 304, the needle 302 is more likely to bend toward a side of the needle 302 including the flat 310. Thus, the needle 302 is received within the lumen 312 such that the flat 310 of the needle 302 is aligned with the first a portion of the wall 314 having the smallest thickness (e.g., the first side 316). In other words, the flat 310 faces toward the first side 316 of the delivery catheter 304.

As the device 100 is inserted through tortuous paths of a body lumen, the needle 302 and delivery catheter 304 flex and bend, as described above, the flat 310 and the offset lumen 312 maintaining alignment with one another. While the device 100 is being inserted to the target location, the bends along the needle 302 correspond to bends along the delivery catheter 304 so that the needle 302 is held in the bent configuration via an interior surface 322 of the delivery catheter 304. Since the delivery catheter 304 and the needle 302 are bent toward the first side 316 and a side of the needle 302 including the flat 310, respectively, a side of the needle 302 opposite the portion including the flat 310 is in contact with the interior surface 322 of the delivery catheter 304 keeping the tissue piercing tip 306, which faces the first side 316 of the delivery catheter 304 away from the interior surface 322 of the delivery catheter 304 to prevent damage thereto. Thus, as the needle 302 is moved distally out of the delivery catheter 304 upon reaching the target tissue, the tip 306 does not pierce the delivery catheter 304, preventing clogging of the channel 308 and//or damage to the needle 302.

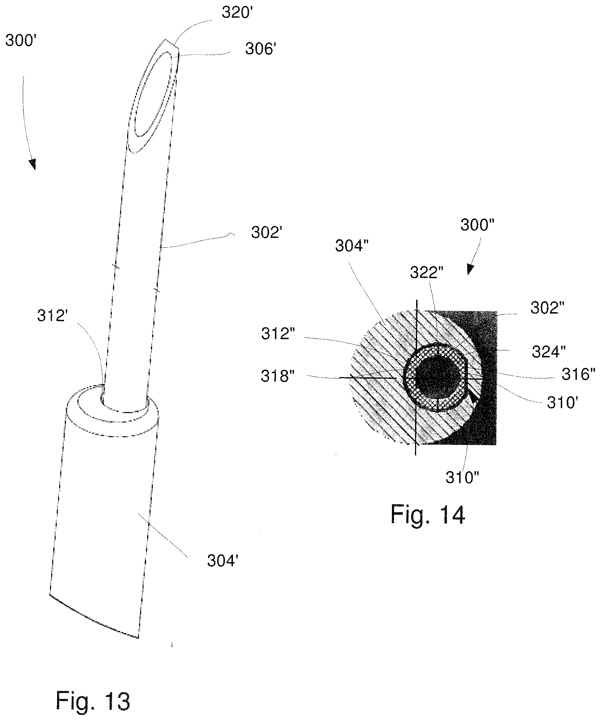

As shown in Fig. 13, a device 300' according to another exemplary embodiment is substantially similar to the device 300, described above, comprising a needle 302' passed through an offset lumen 312' of a delivery catheter 304'. A flat 310' extending along the needle 302', however, extends to a distal-most end of a tip 306' of the needle 302' so that a distal end 320' of the flat 310' is not longitudinally separated from the distal-most end of the tip 306'. Thus, the sharpened tissue piercing tip 306 is angled to a point and is further prevented from piercing the delivery catheter 304'.

As shown in Fig. 14, a device 300" according to yet another exemplary embodiment is substantially similar to the devices 300, 300' described above. The device 300" similarly comprises a needle 302" passed through an offset lumen 312" of a delivery catheter 304". The delivery catheter 304", however, further include a flat 324" along a portion of an interior surface 322" thereof to correspond to a flat 310" of the needle 302". The flat 324" extends along a first side 316" of the needle 302" which has a smaller wall thickness that a second side 318" of the needle 302" substantially opposing the first side 316". Thus, the flats 310" and 324" of the needle 302" and the delivery catheter 304", respectively, further maintain an alignment between the delivery catheter 304" and the needle 302" by preventing relative rotation therebetween.

The present invention is set out in the appended claims. The embodiments, aspects or examples according to the present description that do not fall within the scope of said claims are provided for illustrative purposes only and do not form part of the present invention.

## Claims

1. A needle device (100, 200) for obtaining a tissue sample, comprising:
a distal member (102) extending longitudinally from a proximal end (110) to a distal end (112) and including a distal channel (114) extending longitudinally therethrough, the distal member (102) formed of a first material selected to permit flexing of the distal member (102);
a proximal member (104) extending longitudinally from a proximal end to a distal end (130) and including a proximal channel (132) extending longitudinally therethrough, the proximal member (104) formed of a second material selected to provide axial and torsional strength therealong; and
a coupler (106) for connecting the distal member (102) and the proximal member (104), the coupler (106) extending longitudinally from a proximal end (134) configured to engage the distal end (130) of the proximal member (104) to a distal end (136) configured to engage the proximal end (110) of the distal member (102); and
**characterized in that** the distal member (102) includes an outer sleeve (116) and an inner sleeve (118), the inner sleeve (118) press fit into the outer sleeve (116) to support the outer sleeve (116) during a flexing thereof.

2. The device of claim 1, wherein the distal member (102) includes a plurality of slots extending laterally thereinto form an exterior surface thereof to an interior surface of the distal channel (114), the plurality of slots positioned therealong to permit a desired flexing thereof.

3. The device of any one of claims 1 and 2, wherein the distal member (102) is at least partially formed of Nitinol.

4. The device of any one of claims 1 to 3, wherein the proximal member (104) is formed of stainless steel.

5. The device of any of claims 1-4, wherein the outer sleeve (116) is formed of stainless steel and the inner sleeve (118) is formed ofNitinol.

6. The device of any one of claims 1 to 5, wherein the proximal end (134) of the coupler (106) is configured to be received within the proximal channel (132) of the proximal member (104).

7. The device of any one of claims 1 to 6, wherein the distal end (136) of the coupler (106) is configured to be mounted over the proximal end (110) of the distal member (102).

8. The device of any one of claims 1 to 7, wherein the coupler (106) is welded to the distal and proximal members (102, 104).

9. The device of any one of claims 1 to 8, further comprising a filter (208, 208') positionable within a coupler channel (138) proximally of the proximal end (110) of the distal member (102) to prevent passage of a tissue sample proximally therepast while permitting passage therethrough of one of a vacuum force and a flushing fluid.

10. The device of claim 9, wherein the filter (208, 208') is formed via one of molding valves, spinning fibers, blown fibers and ceramics.

11. The device of any one of claims 9 and 10, wherein a central portion (254, 254') of the filter (208, 208') is configured to receive a flushing fluid therethrough and radially extending outer portions (256, 256') are configured to receive a vacuum force therethrough.

## Patentansprüche

1. Nadelvorrichtung (100, 200) zum Erhalten einer Gewebeprobe, die aufweist:
ein distales Teil (102), das sich von einem proximalen Ende (110) längs zu einem distalen Ende (112) erstreckt und einen sich längs dadurch erstreckenden distalen Kanal (114) aufweist, wobei das distale Teil (102) aus einem ersten Material gebildet ist, das so ausgewählt ist, dass es Biegung des distalen Teils (102) ermöglicht;
ein proximales Teil (104), das sich von einem proximalen Ende längs zu einem distalen Ende (130) erstreckt und einen sich längs dadurch erstreckenden proximalen Kanal (132) aufweist, wobei das proximale Teil (104) aus einem zweiten Material gebildet ist, dass so ausgewählt ist, dass es für Axial- und Torsionsfestigkeit daran entlang sorgt; und
einen Koppler (106) zum Verbinden des distalen Teils (102) und des proximalen Teils (104), wobei sich der Koppler (106) von einem proximalen Ende (134), das so konfiguriert ist, dass es einen Eingriff mit dem distalen Ende (130) des proximalen Teils (104) herstellt, längs zu einem distalen Ende (136) erstreckt, das so konfiguriert ist, dass es einen Eingriff mit dem proximalen Ende (110) des distalen Teils (102) herstellt; und
**dadurch gekennzeichnet, dass**
das distale Teil (102) eine Außenhülse (116) und eine Innenhülse (118) aufweist, wobei die Innenhülse (118) in die Außenhülse (116) eingepresst ist, um die Außenhülse (116) während einer Biegung davon zu stützen.

2. Vorrichtung nach Anspruch 1, wobei das distale Teil (102) mehrere Schlitze aufweist, die sich darin seitlich von einer Außenfläche davon zu einer Innenfläche des distalen Kanals (114) erstrecken, wobei die mehreren Schlitze daran entlang positioniert sind, um eine gewünschte Biegung davon zu ermöglichen.

3. Vorrichtung nach Anspruch 1 oder 2, wobei das distale Teil (102) mindestens teilweise aus Nitinol gebildet ist.

4. Vorrichtung nach einem der Ansprüche 1 bis 3, wobei das proximale Teil (104) aus Edelstahl gebildet ist.

5. Vorrichtung nach einem der Ansprüche 1 bis 4, wobei die Außenhülse (116) aus Edelstahl gebildet ist und die Innenhülse (118) aus Nitinol gebildet ist.

6. Vorrichtung nach einem der Ansprüche 1 bis 5, wobei das proximale Ende (134) des Kopplers (106) so konfiguriert ist, dass es im proximalen Kanal (132) des proximalen Teils (104) aufgenommen ist.

7. Vorrichtung nach einem der Ansprüche 1 bis 6, wobei das distale Ende (136) des Kopplers (106) zur Montage über dem proximalen Ende (110) des distalen Teils (102) konfiguriert ist.

8. Vorrichtung nach einem der Ansprüche 1 bis 7, wobei der Koppler (106) mit dem distalen und proximalen Teil (102, 104) verschweißt ist.

9. Vorrichtung nach einem der Ansprüche 1 bis 8, ferner mit einem Filter (208, 208'), das in einem Kopplerkanal (138) proximal vom proximalen Ende (110) des distalen Teils (102) positionierbar ist, um proximalen Durchgang einer Gewebeprobe daran vorbei zu verhindern, während es Durchgang einer Vakuumkraft oder eines Spülfluids ermöglicht.

10. Vorrichtung nach Anspruch 9, wobei das Filter (208, 208') über Formventile, Spinnfasern, Blasfasern oder Keramikmaterialien gebildet ist.

11. Vorrichtung nach Anspruch 9 oder 10, wobei ein Mittelabschnitt (254, 254') des Filters (208, 208') so konfiguriert ist, dass er ein Spülfluid dadurch aufnimmt, und sich radial erstreckende Außenabschnitte (256, 256') so konfiguriert sind, dass sie eine Vakuumkraft dadurch aufnehmen.

## Revendications

1. Dispositif à aiguille (100, 200) pour obtenir un échantillon de tissu, comprenant :
un élément distal (102) qui s'étend longitudinalement depuis une extrémité proximale (110) jusqu'à une extrémité distale (112) et incluant un canal distal (114) qui s'étend longitudinalement dans celui-ci, l'élément distal (102) formé d'un premier matériau choisi pour permettre la flexion de l'élément distal (102) ;
un élément proximal (104) qui s'étend longitudinalement depuis une extrémité proximale jusqu'à une extrémité distale (130) et incluant un canal proximal (132) qui s'étend longitudinalement dans celui-ci, l'élément proximal (104) formé d'un second matériau choisi pour conférer une résistance axiale et à la torsion le long de celui-ci ; et
un organe d'accouplement (106) pour relier l'élément distal (102) et l'élément proximal (104), l'organe d'accouplement (106) s'étendant longitudinalement depuis une extrémité proximale (134) configurée pour coopérer avec l'extrémité distale (130) de l'élément proximal (104) jusqu'à une extrémité distale (136) configurée pour coopérer avec l'extrémité proximale (110) de l'élément distal (102) ; et
**caractérisé en ce que** l'élément distal (102) inclut un manchon externe (116) et un manchon interne (118), le manchon interne (118) ajusté par ajustage serré dans le manchon externe (116) pour soutenir le manchon externe (116) pendant une flexion de celui-ci.

2. Dispositif selon la revendication 1, où l'élément distal (102) inclut une pluralité de fentes qui s'étendent latéralement dans celui-ci depuis une surface extérieure de celui-ci jusqu'à une surface intérieure du canal distal (114), la pluralité de fentes positionnée le long de celui-ci pour permettre une flexion souhaitée de celui-ci.

3. Dispositif selon l'une quelconque des revendications 1 et 2, où l'élément distal (102) est formé au moins partiellement de Nitinol.

4. Dispositif selon l'une quelconque des revendications 1 à 3, où l'élément proximal (104) est formé d'acier inoxydable.

5. Dispositif selon l'une quelconque des revendications 1-4, où le manchon externe (116) est formé d'acier inoxydable et le manchon interne (118) est formé de Nitinol.

6. Dispositif selon l'une quelconque des revendications 1 à 5, où l'extrémité proximale (134) de l'organe d'accouplement (106) est configurée pour être reçue dans le canal proximal (132) de l'élément proximal (104).

7. Dispositif selon l'une quelconque des revendications 1 à 6, où l'extrémité distale (136) de l'organe d'accouplement (106) est configurée pour être montée au-dessus de l'extrémité proximale (110) de l'élément distal (102).

8. Dispositif selon l'une quelconque des revendications 1 à 7, où l'organe d'accouplement (106) est soudé aux éléments distal et proximal (102, 104).

9. Dispositif selon l'une quelconque des revendications 1 à 8, comprenant en outre un filtre (208, 208') pouvant être positionné dans un canal d'organe d'accouplement (138) de manière proximale par rapport à l'extrémité proximale (110) de l'élément distal (102) pour empêcher le passage d'un échantillon de tissu de manière proximale devant celui-ci tout en permettant le passage à travers celui-ci de l'un d'une force de vide et d'un fluide de balayage.

10. Dispositif selon la revendication 9, où le filtre (208, 208') est formé via l'un de valves de moulage, fibres de filage, fibres soufflées et céramique.

11. Dispositif selon l'une quelconque des revendications 9 et 10, où une partie centrale (254, 254') du filtre (208, 208') est configurée pour recevoir un fluide de balayage à travers celle-ci et des parties externes (256, 256') qui s'étendent radialement sont configurées pour recevoir une force de vide à travers celles-ci.
